(19) **Europäisches Patentamt / European Patent Office / Office européen des brevets**

(11) **EP 2 075 754 A1**

(12) **EUROPEAN PATENT APPLICATION**
published in accordance with Art. 153(4) EPC

(43) Date of publication:
**01.07.2009 Bulletin 2009/27**

(21) Application number: **07828314.0**

(22) Date of filing: **14.09.2007**

(51) Int Cl.:
***G06Q 50/00*** *(2006.01)*

(86) International application number:
**PCT/JP2007/068481**

(87) International publication number:
**WO 2008/032875 (20.03.2008 Gazette 2008/12)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **15.09.2006 JP 2006250798**

(71) Applicant: **Keio University**
**Tokyo 108-8345 (JP)**

(72) Inventors:
• **SUEMATSU, Makoto**
**Tokyo 160-8582 (JP)**
• **KAWAHARA, Hitoshi**
**Chiba-shi, Chiba 266-0032 (JP)**

(74) Representative: **Nicholls, Michael John**
**J.A. Kemp & Co.**
**14 South Square**
**Gray's Inn**
**London**
**WC1R 5JJ (GB)**

(54) **PLATELET THROMBUS FORMATION SIMULATOR**

(57) A platelet thrombus formation simulator, said simulator equipped with the following means:
(a) a means that selects, from previously stored equations, an equation for the computation of adhesion force according to parallel activation grade;
(b) a means that calculates the platelet adhesion force based on the equation depending on the activation grade of each platelet; and
(c) a means that outputs the coagulation condition of platelets based on the calculated adhesion force of each platelet.

Figure 9

**Description**

TECHNICAL FIELD

The present invention relates to a platelet thrombus formation simulator for modeling *in vivo* platelet formation, and a computer program used to simulate the formation of platelet thrombi.

BACKGROUND ART

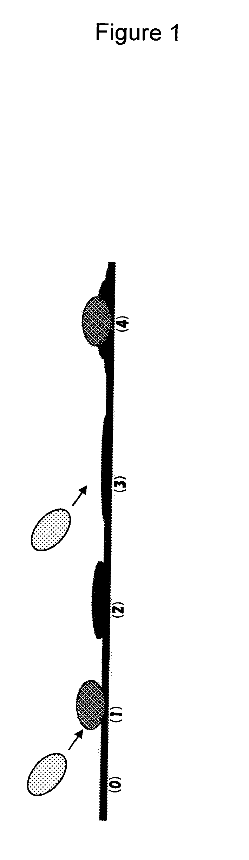

According to recent biochemical research results, the mechanisms that cause the process through which platelet thrombi form inside blood vessels is as shown in Fig. 1.

The platelet (Fig. 1 (0)) recognizes von Willebrand factor (hereinafter referred to as "vWF") expressed at the vascular damage location and on the surface of the vascular endothelial cells and begins a reversible adhesion reaction via the GPIbα complex (Fig. 1 (1)). Since a certain amount of vWF exists in blood, it is also possible for a plurality of platelets to adhere to each other through the use of vWF. As a result, the platelets rapidly adhere transiently to the injured wall, even in a rapid flow, and migration speed is reduced. This adhesion phenomenon allows the platelets to remain in contact with the blood vessel wall. During this period, the activation signal enters the cell interior showing adhesion reaction, causing GPIIb/IIIa complex expression and a multistep, functional activation with a conformation reaction between at least three different complexes, thereby strengthening adhesion (Fig. 1 (2)). Subsequently, the adhesiveness of the GPIIb/IIIa complex is stimulated by platelet activation reactions occurring in tandem, completing the adhesion reaction at the wall in resistance to the flow (Fig. 1 (3)). With the vWF expressed at the stabilized adhered platelets as a target, platelets are continuously replenished by the same mechanism described above, forming a platelet thrombus (platelet aggregate) (Fig. 1 (4)). Here, the platelet adhesion force in Fig. 1 (0) to (2) is strongly influenced by the shear rate of plasma flow. In this way, platelet thrombi form through multiple activation steps.

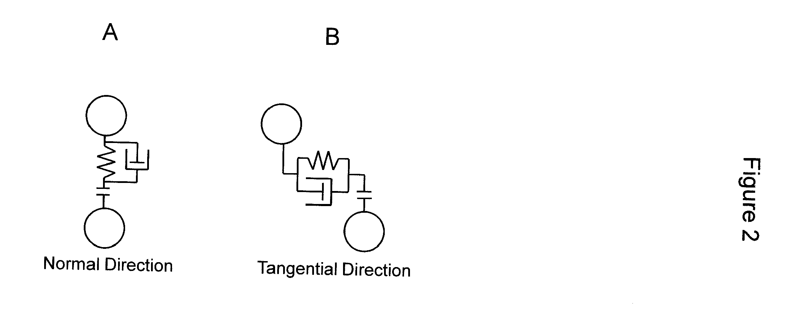

In conventional models of the above process of platelet thrombus formation, platelets are assumed to be spheres, as in Fig. 2, and when the distance between spheres meets or falls below a predetermined distance, a certain normal direction spring (Fig. 2 (A)) and tangential direction spring (Fig. 2 (B)) are positioned (Miyazaki H, and Yamaguchi T. Formation and destruction of primary thrombi under the influence of blood flow and von Willebrand factor analyzed by a discrete element method. Biorheology 2003, vol 40, 265-272.).

Conventional models, however, assume that interplatelet adhesion is constant and not influenced by activity level and flow. As a result, conventional models have the following problems:

(i) Can not account for changes in adhesion force caused by the shear stress of platelet flow;
(ii) Can not account for the effects of a plurality of adhesion molecules associated with platelet activation;
(iii) Can not account for the interaction between multiple platelets of different activity levels.

Accordingly, calculations using conventional models cannot account for the biological processes of platelet thrombus formation, making it exceedingly difficult to realistically simulate the platelet thrombus formation process that occurs *in vivo*.

In addition, it is necessary to directly modify the calculation program when changes are made in interplatelet adhesion force, presenting the drawback that individuals not familiar with the program can not perform calculations or virtual experiments.

Furthermore, to display the results of calculations, it is necessary to display the platelet adhesion process status and plasma flow rate change, which cannot be displayed on the same screen, separately. Accordingly, it is cumbersome and complicated to control the display of calculation results. Also, conventional models do not take the functions of a plurality of adhesion molecules into account, making it impossible to ascertain the platelet adhesion process and time-series changes in scattering frequency.

Thus, conventional models do not take into account changes in adhesion force due to plasma flow shear rate and the functions of the plurality of adhesion molecules that platelets possess. Conventional models, in other words, can only account for processes (0) and (1) in Fig. 1.

DISCLOSURE OF THE INVENTION

It is an object of the present invention to provide a platelet thrombus formation simulator that mechanically models the platelet formation that actually occurs *in vivo*, and a program that allows said simulator to function.

As a result of assiduous research intended to overcome these disadvantages, the present inventors perfected the present invention by developing a mathematical model for the platelet thrombus formation process and discovering that said model could be used to almost perfectly simulate actual measurements of the platelet thrombus formation process.

More specifically, the present invention is:

(1) A platelet thrombus formation simulator equipped with the following means:

(a) a means for selecting, from pre-stored equations, an equation for the calculation of adhesion force corresponding to platelet activation grade;
(b) a means for calculating the platelet adhesion force based on the equation in accordance with platelets in each activation grade; and
(c) a means for outputting the platelet coagulation status based on the calculated adhesion force of each platelet.

In the present invention, the procedure wherein the inputted parameters for the thrombus detachment of the platelets are used to calculate the shear stress applied to the surface of the coagulated platelet aggregates and then output the detachment status of platelets from the platelet aggregates based on the shear stress can also be included.
The abovementioned equation uses parameters related to thrombi formation.
Further, the abovementioned parameters are at least one of the following selected from the group consisting of: concentration of platelets in the blood, platelet diameter, platelet concentration, platelet distribution ratio in blood vessel, spring coefficient, blood vessel diameter, blood vessel length, artery and vein classification, blood flow rate, size or shape of injured location, and type and density of adhesion molecules expressing at the injured location.
In the simulator of the present invention, it is preferable that the output of the abovementioned platelet coagulation status and/or detachment status is output to a predetermined display means. In this instance, the display means is at least one of the following selected from the group consisting of: an animated image depicting the abovementioned platelet coagulation status and/or detachment status; a display of the activation level of individual platelets constituting the abovementioned platelet aggregates; an animation depicting plasma shear rate distribution; a display of the scattering frequency of the abovementioned platelet aggregates and the size of each cluster; or a graph depicting the activation level of individual platelets included in the scattered aggregates.
The display output in accordance with the abovementioned output means is at least one of the following selected from the group consisting of: coagulation status and/or detachment status on a perspective view of blood vessels; coagulation status and/or detachment status on a longitudinal cross-section diagram of blood vessels; and a longitudinal shear stress distribution diagram of blood vessels.
The abovementioned parameter related to platelet thrombus separation is at least one of the following selected from the group consisting of: platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.
(2) The program for the simulation of platelet thrombus formation, said program executing the following procedure in a computer:

(a) selecting, from previously stored equations, an equation for the computation of adhesion force in accordance with platelet activation grade;
(b) calculating the platelet adhesion force based on the equation corresponding to platelets in each activation grade;
(c) outputting the coagulation status of platelets based on the calculated adhesion force of each platelet.

The program of the present invention can also include the procedure wherein the inputted parameters related to platelet thrombus detachment are used to calculate the shear stress exerted on coagulated platelets, and the detachment state from the platelet aggregate is output on the basis of the aforementioned shear stress.
The abovementioned equation uses a parameter related to thrombus formation.
The abovementioned parameter is at least one of the following selected from the group consisting of: concentration of platelets in the blood, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.
It is desirable for the output of the platelet coagulant status and/or detachment status to be output to the designated display means. In this instance, the display means is at least one of the following selected from the group consisting of: an animated image of the platelet coagulant status and/or detachment status; a display of the activation level of individual platelets constituting the platelet aggregates; an animation depicting platelet shear rate distribution; a display of the scattering frequency of the platelet aggregates and the size of each cluster; and a graph depicting the activation level of individual platelets included in the scattered aggregates.

Further, the display output according to the abovementioned output means is at least one of the following selected from the group consisting of: coagulation status and/or detachment status on a perspective view of blood vessels; coagulation status and/or detachment status on a longitudinal cross-section diagram of blood vessels; and a longitudinal shear stress distribution diagram of blood vessels.

The abovementioned platelet thrombus separation-related parameter is at least one of the following selected from the group consisting of: platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.

(3) A computer-readable recording medium on which the computer program in (2) above is stored.

The present invention provides a platelet thrombus formation simulator, a computer program for simulating thrombus formation, and the recording medium upon which said program is recorded.

(1) The simulator of the present invention makes it possible to reproduce platelet thrombus formation processes in accordance with platelet coagulation reactions that actually occur *in vivo.* Of the adhesion molecules, the adhesion force of GPIbα changes notably due to wall shear rate. In order to reflect this information, the present invention is equipped with a means for calculating shear stress in real time and a means for displaying the results of the calculations. These means are an indispensible element lacking in preceding simulations and are one major characteristic of the present invention.

(2) The simulator of the present invention uses a GUI (Graphical User Interface) to set calculation conditions, making it easy to configure calculation condition settings in accordance with the user's wishes. More specifically, since calculation condition input and result output can be performed on a single screen, even a person not familiar with the calculations can use the simulator. Since the calculation conditions and calculation results are saved in a database, parameter surveys in which conditions have been partially modified can be set easily and in a short period of time.

(3) In the simulator of the present invention, an injured location with the desired shape and desired expressed quantity of collagen and vWF can be set inside a virtual vascular endothelial cell. Consequently, it is also possible to simulate a damage model in which only vascular endothelial cells are damaged by hemorrhagic disorders that pierce the vascular walls, liquid factors and toxins in the blood, hindrance factors, etc., and collagen is not exposed.

(4) The simulator of the present invention can calculate the effects of the plurality of adhesion molecule functions possessed by platelets and can therefore be applied to the development of antiplatelet agents that take into account the influence of each molecule. For example, blood samples are taken from patients before and after administration of an antiplatelet agent, the "spring coefficient" for each adhesion molecule is estimated from the resulting platelet function test data, and by inputting this data, the platelet dissolution and destruction process before and after the administration of the drug can be predicted.

(5) The characteristics described in (4) can be simulated by conducting an *in vitro* function test to measure in advance the adhesion molecule inhibitory effect of GPIbα, GPIIb/IIIa, etc. for an antiplatelet agent not yet in practical use and inputting the results into the spring coefficient of the adhesion molecule. By so doing, a simulation can be made that predicts the thrombus inhibitory effect and "flying thrombus" inhibitory effect a specific antiplatelet agent will exhibit for blood vessel shapes of various sites within the human body and under blood flow rate conditions.

(6) The characteristics described in (4) can also be used to distinguish between existing antiplatelet agents. For example, the differences in thrombus inhibition effect when aspirin and other antiplatelet agents are administered can be compared and contrasted. More specifically, it is thought that aspirin, which is a cyclooxygenase inhibitor, inhibits the process in Grades 3 to 5 (an explanation of Grades is provided hereinafter) by inhibiting thromboxane A2 in the platelet activation process. By contrast, cilostazol, a phosphodiesterase inhibitor, increases cyclic AMP inside platelet cells, thus inhibiting the Grade 1 to 2 process that is related to shear stress-dependent platelet coagulation in the platelet activation process. Differences between the effects of these drugs can be compared in the present invention. Consequently, the ways in which these two drugs inhibit platelet adhesion and break down thrombi can be clarified.

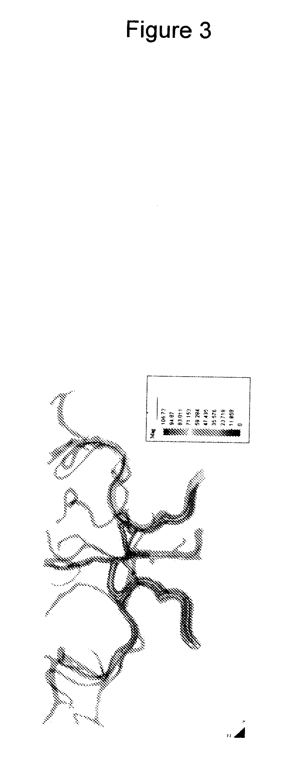

(7) The simulator of the present invention can also be applied to complex shapes, thereby enabling ascertainment of the state of platelet aggregate scatter in complex vascular networks. For example, after constructing a vascular network based on a vascular cast specimen of an animal brain or a human blood vessel shape extracted from the CT or the MRI, a prediction of obstruction due to platelet aggregate can be made based on the constructed vascular network or blood vessel shape (Fig. 3). Fig. 3 shows an example of platelet aggregates being transported even in delicate blood vessels, thereby showing results simulated by the simulator of the present invention.

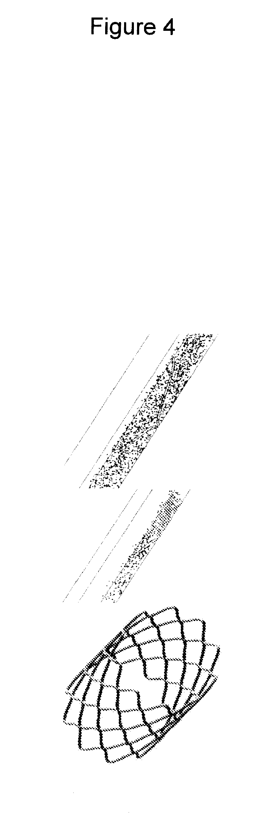

(8) In addition, the process through which stents installed in blood vessels cause thrombus formation can be recreated, enabling optimization of stent shapes (Fig. 4.)

The present invention is explained in detail below. The following embodiment is an illustrative example provided to explain the present invention and is not intended to limit the present invention to this embodiment. The present invention can be implemented in a variety of forms as long as said forms do not deviate from the essence of the invention.

All printed publications cited in this specification, for example prior art documents and Kokai publications, Patent Gazettes and other patent literature are, in their entirety, incorporated into this specification as reference. This specification includes the content of Japan patent application specification 2006-250798, which forms the basis for the priority claim in this specification.

BRIEF DESCRIPTION OF THE DRAWINGS

Fig. 1 shows the platelet thrombus formation mechanisms.

Fig. 2 shows interplatelet adhesion models using a normal direction spring and a tangential direction spring.

Fig. 3 shows intracerebral artery platelet scatter status (the trajectory of a platelet aggregate moving in an intracerebral artery).

Fig. 4 shows an example of a thrombus formation process calculation in accordance with stent shape (calculation example wherein the size of the stent mesh has been changed).

Fig. 5 is a pattern diagram of platelets in quiescent and activated states.

Fig. 6 is a pattern diagram of the thrombus formation process.

Fig. 7 is a pattern diagram of platelet activation states.

Fig. 8 is a pattern diagram of springs with different spring coefficients.

Fig. 9 is a configuration diagram of the present invention.

Fig. 10 shows the input screen of the calculation condition setting unit.

Fig. 11 is a detailed configuration diagram of the system that executes the program of the present invention.

Fig. 12 is a flowchart explaining how the program of the present invention functions.

Fig. 13 is a diagram showing calculation results.

Fig. 14 is a diagram showing calculation results.

Fig. 15 is a diagram showing calculation results.

EXPLANATION OF SYMBOLS

100: system, 910: calculation unit, 920: database, 101: control unit, 102: transmission/reception unit, 103: input unit, 104: output unit, 105: ROM, 106: RAM, 107: hard disk drive, 108: CD-ROM drive, 111: internet cable, 120: CD-ROM

BEST MODE FOR CARRYING OUT THE INVENTION

1. Summary

The present invention is a platelet thrombus formation simulator that was perfected by modeling the functions of a plurality of adhesion molecules in individual platelets through the use of Voigt models in which springs and dashpots are used to model the interactions between platelets or between a platelet and vWF and/or collagen.

The simulator of the present invention reproduces the actual *in vivo* phenomenon in which the probability of contact between platelets and vascular walls fluctuates due to wall hemodynamics by reproducing, in a simulation, the unevenness of endovascular platelet distribution that changes due to red blood cell occupation of the main flow caused by increase of blood flow speed. Simulation is based on actual experiment data for single-cell behavior analysis from an ultra high-speed biological fluorescence microscope. The present invention also makes it possible to describe the effects of platelet collision and adhesion in terms of the nonlinear spring coefficient and attenuation coefficient, in other words, display as animation the change in platelet adhesion force that occurs when shear stress changes the spring coefficient in the horizontal and vertical directions.

The simulator of the present invention is a platelet thrombus formation simulator that detects the shear stress caused by the plasma flow shear rate in microvessels by taking into account the functions of the plurality of adhesion molecules that platelets actually have *in vivo,* uses the interaction between adhesion molecules that changes the adhesion force of platelets, and enables the display of the hemostatic process in the form of three-dimensional animation based on shear stress data and platelet adhesion force data.

The simulator of the present invention makes it possible to quantitatively evaluate the shear stress caused by plasma flow and frequency of platelet aggregate appearance according to platelet activation state or the scattering frequency thereof (frequency with which platelet thrombus clusters detach from the site of hemostasis). After comparing the platelet adhesion process data calculated by the simulator of the present invention to the platelet adhesion process data derived from experimentation, it is possible to evaluate the platelet coagulation status of the endovascular damage location by performing calculations that change shear stress and activation state.

The present invention is a program for the simulation of platelet thrombus formation and can simulate platelet formation with regards to the desired fluid channel and fluid.

The specific simulation method is as follows: the endovascular space is divided into grid-shaped sections; for each divided section, a discrete equation of continuity and discrete Navier-Stokes equations are used to perform a computation each infinitesimal time step/interval and calculate a numerical value of the motion elements. Then, platelet changes can be analyzed by integrating the computation results of each of these grid-shaped sections and simulating plasma flow in the entirety of the blood vessel. It is possible to analyze changes in the flow of plasma in a given time span by letting time proceed for infinitesimal time steps/intervals and evaluating the equations by infinitesimal time step/interval when analyzing changes in platelets that accompany the passage of time or flows of plasma in different time spans.

The distribution of pressure exerted on plasma flow can be made visible by converting the values obtained from the abovementioned computation results into an isobaric line or color.

2. Mathematical Model and Platelet Thrombus Simulator

The present invention consists of the following 2 elements:

· A mathematical model of the platelet thrombus formation process
· A platelet thrombus simulator that uses the abovementioned mathematical model These elements are explained below.

(1) Platelet thrombus formation process mechanism

In order to explain the mathematical model of the platelet thrombus formation process, the mechanism of platelet thrombus formation is explained first.

Platelets, using three basic adhesion molecules (GPIb$\alpha$, GPIaIIa, and GPIIb/IIIa), adhere through a multistep process comprising:

(i) a "rolling reaction" called "tethering" in the blood,
(ii) an adhesion reaction called "adhesion",
(iii) to (iv) : initiating morphological change and a release reaction, adhering irreversibly, adhering to other platelets and ending in a coagulation reaction. This process is explained further below.

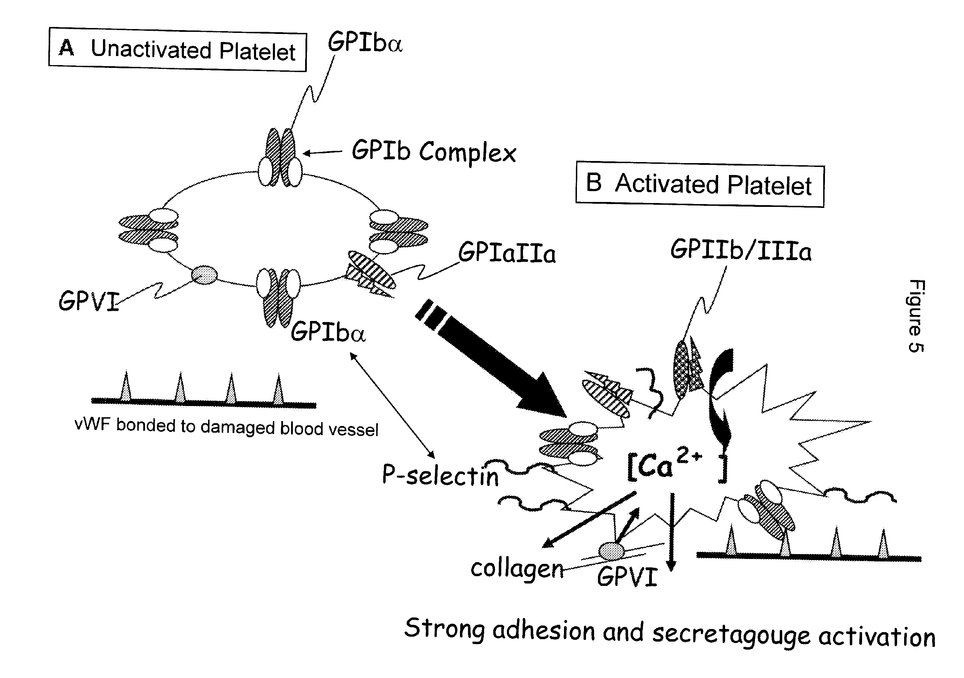

In a normal blood flow state in which there is no vascular damage, platelets are not activated (quiescent), and express glycoproteins called GPIb$\alpha$ and GPIaIIa in addition to a collagen receptor called GPVI on their cell membrane surface (Fig. 5A). If vascular damage occurs, platelets in a quiescent state enter an activated state in order to form thrombi (Fig. 5B).

The abovementioned platelets in a quiescent state are in "Grade 0". In the present invention, the term "Grade" is used to express the activation state of platelets in accordance with the type of molecules on the platelet's surface and the strength of the adhesion force. The term "state" encompasses a range of conditions including those that are momentary and those that persist for a certain period of time.

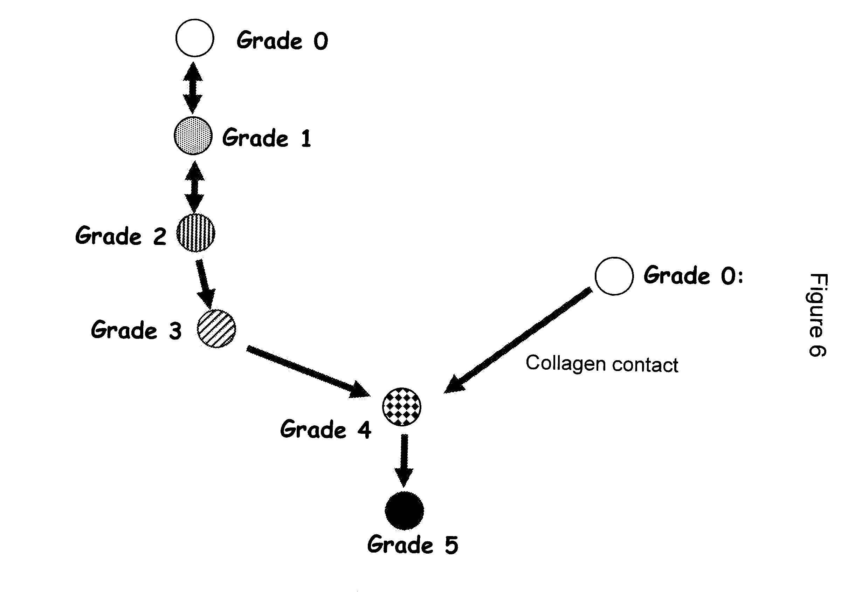

The activation process of individual platelets is explained in detail below (Fig. 6).

Grade 0 platelets are inactive. If blood vessel damage occurs, vWF is secreted at the injured location and the Grade 0 platelet (Fig. 6) binds with vWF through the GPIb$\alpha$ complex, forming a string of two platelets called a "tether", thereby becoming a Grade 1 platelet (Fig. 6). In other words, Grade 1 is the step at which adhesion of GPIb$\alpha$ with vWF begins. Grade 1 platelets become Grade 2 platelets by binding with a plurality of GPIb$\alpha$ via other platelets (Fig. 6). In other words, Grade 2 is the state in which adhesion occurs in a plurality of molecule sets transmitted by GPIb$\alpha$ and intracellular signaling occurs. These reactions between Grades are reversible. The platelet activation process can revert if platelets separate due to physical stress such as flow at the wall. It is known that the adhesion force of vWF and GPIb$\alpha$ is increased by wall shear stress. In the present invention, a program was designed to enable the configuration of a simulation that reflects actual measurement data to meet user needs in accordance with data from actual experiments that were performed using a rotating disk-shaped platelet aggregation analysis device etc.

Since intracellular signals for Grade 2 platelets are input, GPIIb/IIIa activation occurs after a fixed period of time elapses. It is known that three different steric structures with different adhesion properties exist in the GPIIb/IIIa activation state. The activation state associated with the structure with the weakest adhesion force is defined as Grade 3, while Grade 4 and Grade 5 are defined as the activation states associated with intermediate and strong adhesion force, respectively. By contrast, although an adhesion molecule called "GPVI" exists on the platelet membrane (Fig. 5), the process is known wherein collagen, one of the extracellular matrices, contacts GPVI, thereby activating IP3-mediated intracellular signal transmission, and directly causing GPIIb/IIIa confirmation change. In the present invention, a program is introduced for instances in which this process triggers the Grade 4 activation process.

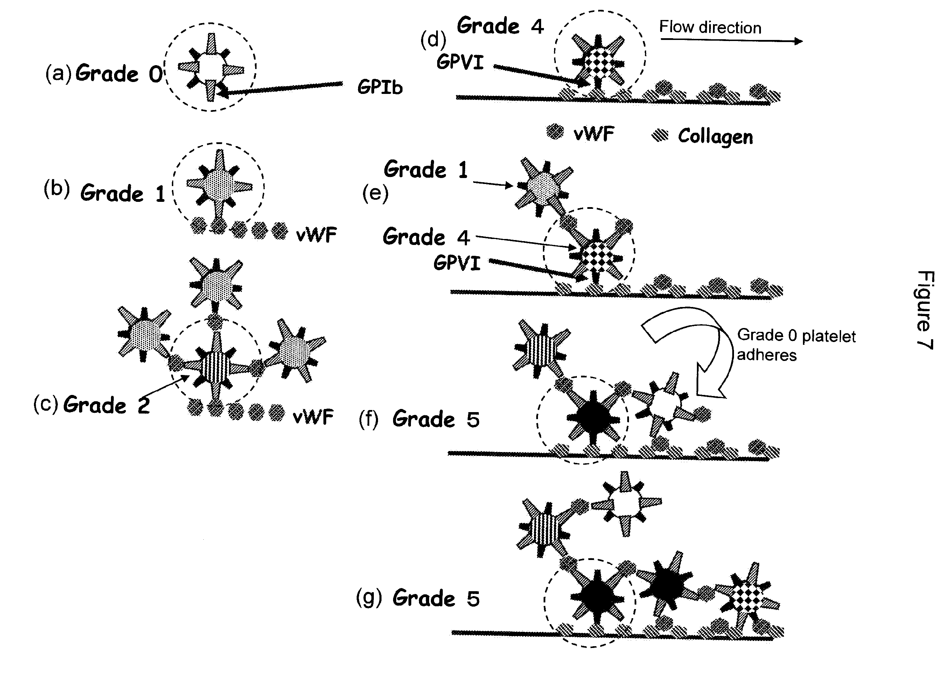

Fig. 7 shows a conceptual diagram of a model that illustrates how platelets in different activation states (Grades) form an aggregate when a plurality of platelets interact according to the activation process shown in Fig. 6. When Grade 0 platelets bind with the vWF that exists in blood and at the injured surface, this reaction triggers the formation of small aggregates formed from a plurality of platelets (Fig. 7 a, b, c). When collagen is exposed at the injured surface, GPVI-mediated activation occurs, causing the contacted platelets to transition to Grade 4 (Fig. 7d). Here, if other platelets flow in and make contact, and a signal enters due to adhesion mediated by a plurality of GPIb$\alpha$ with the Grade 4 platelets, the activation level rises, causing Grade 4 platelets to transition to Grade 5, and Grade 1 platelets to transition to Grade 2 (Fig. 7e, f). If platelets make accidental contact with collagen due to the effects of the flow, the activation level reaches level 4, causing platelets with a high activation level (Grade 5) to gradually aggregate (Fig. 7f, g). In contrast, new Grade 0 platelets additionally adhere to the surface layer, forming an aggregate.

Although the present invention does not take the effects of other blood-coagulating factors into account, it was assumed that platelets that have reached Grade 5 (Fig. 7g) will not separate from the adhesion surface under any flow conditions. Platelets exhibited an irreversible secretory response *in vivo*, which was considered to be the influence of a strong hemostasis process coupled with fibrin formation at the wall. In addition, although the present invention does not take into account the effects of other adhesion molecules such as P-selectin, it is possible to add P-selectin and other similar adhesion molecules for the purpose of observing their effects.

(2) Platelet Thrombus Formation Process Mathematical Model

Since platelets, as noted above, undergo a multi-step process *in vivo* during the aggregation reaction, in the present invention the 6 steps in the platelet thrombus formation process shown in Fig. 1 were defined as an activated state of simulated platelets in Fig. 6 and Fig. 7, and a mathematical model was developed that changes the adhesion strength of virtual platelets in each stage. In this mathematical model, platelet adhesion strength can also change in accordance with plasma shear rate.

Each step in Fig. 1 can be applied to Figs. 6 and 7 as described below.

Fig. 1 (0): Grades 0 and 1 in Figs. 6 and 7
Fig. 1 (1): Grades 1 and 2 in Figs. 6 and 7
Fig. 1 (2): Grade 3 in Fig. 6
Fig. 1 (3): Grade 4 in Figs. 6 and 7
Fig. 1(4): Grade 5 in Figs. 6 and 7

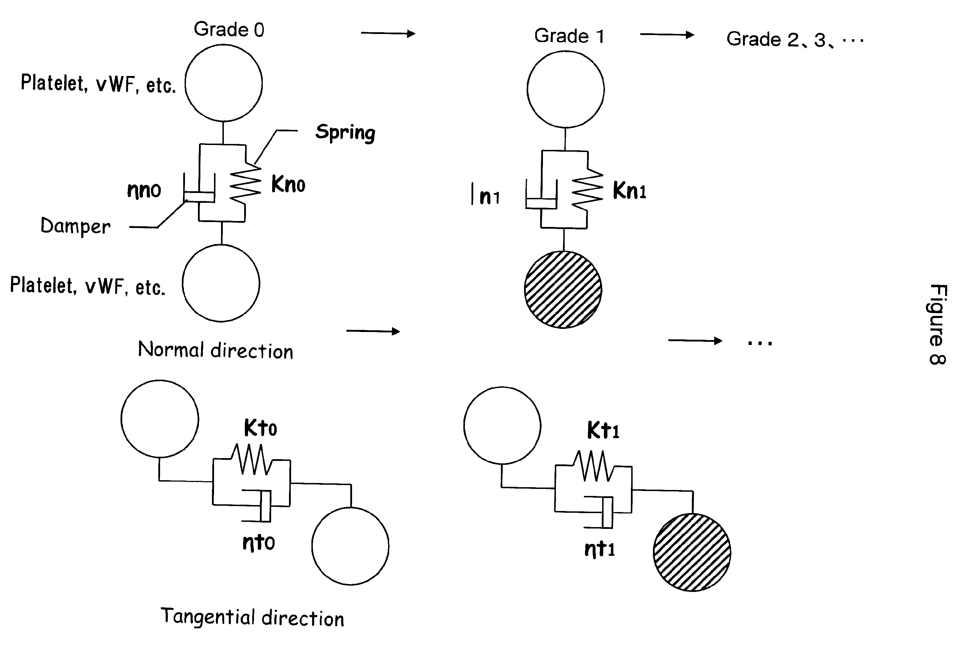

Here, if a single platelet is focused upon as in Fig. 7 (platelet inside the broken circle in Fig. 7), the platelet will, as noted above, pass through a plurality of activation states (Grades) before forming a thrombus and will have a plurality of different adhesion forces in each Grade. Consequently, in the present invention, a plurality of "springs" having different spring coefficients was positioned for each virtual platelet in order to express adhesion forces (Fig. 8). These springs express the adhesion forces of adhesion molecules involved in platelet adhesion. Fig. 8 is a model of rheological objects and shows the following for each Grade: the spring coefficients for the Voigt element Kn0, Kn1...(vertical direction), and Kt0, Kt1,...(horizontal direction); in addition to the viscosity coefficient for the damper element $\eta$n0, $\eta$n1,... (vertical direction), and $\eta$nt0, $\eta$nt1,... (horizontal direction). Then, in the present invention, each spring coefficient is changed for each platelet Grade, and an adhesion pattern that corresponds to actual platelet adhesion is recreated on a computer. Here, the model was designed so that blood vessel diameter and the quantity of platelets that flow within a blood vessel or the shape of a vascular channel could be modified. In the simulator of the present invention, a GUI can be used to modify the shear rate of plasma flow and the platelet adhesion force due to platelet activation, display the platelet adhesion process time-dependently and in three-dimensional space, and to simultaneously display changes in the shear rate of plasma flow at the periphery of the platelet thrombus in real time.

In each platelet Grade, each spring coefficient $K$ and viscosity coefficient $\mu$ in the vertical direction and horizontal direction of the Voigt model can be defined as below.

The viscosity coefficient for Grade 0 is indicated as $\mu_0$, while the spring coefficient is indicated as $K_0$. Coefficients for other Grades are indicated in the same way.

Grade 0:

$$K_0 = 0$$

$$\mu_0 = 0$$

In Grade 0, platelets are not adhered to vWF or other activated platelets, and the spring coefficient used to define adhesion force is 0.

Grade 1:

In Grade 1, the spring coefficient is changed using the following equation depending on the shear stress.

$$K_1 = \frac{1}{2}\rho v^2 A \cdot \left\{ B + C \cdot \tanh(D \cdot S)^2 \right\}$$

Here

ρ: Blood Density
*A, B, C, D* : Each is a positive constant for the fitting of experimental data to the relationship between the fluid force exerted on a single platelet by plasma flow and the adhesion force caused by shear stress. Specifically, *A*=2.4×10⁴, *B*=0.025, *C*=1.0, *D*=0.0017, approximately. However, these numerical values can fluctuate, and are not limited to the numerical values noted above.
*S* : Blood Shear Rate
ν: Plasma Flow Velocity

An increase in plasma flow velocity yields a linear increase in shear stress. Accordingly, when flow velocity is low and when flow velocity is high, it is necessary for platelet adhesion force to increase in accordance with an increase in shear stress in order for platelets to accumulate in the same manner. Further, in order for platelet adhesion to be expedited when flow velocity speeds up (when shear stress is high), the increase in adhesion force must be greater than the increase in shear stress caused by an increase in flow velocity in comparison to when flow velocity is slow (when shear stress is low). The above equation provides an increase in adhesion force caused by shear stress based on the fluid force exerted on a single platelet.

$\frac{1}{2}\rho v^2 A$ is the fluid force exerted by plasma flow on a single platelet.

The following equation is used to solve for viscosity coefficient μ, which suppresses spatter when a platelet contacts another platelet.

$$\mu_1 = 2\sqrt{mK_1}$$

Here

m: Platelet Mass
$K_1$: Spring Coefficient

Incidentally, if the predetermined value is exceeded when calculating the shear stress exerted on the surface of a clump of aggregated platelets, in other words, if the distance between platelets meets or exceeds the value that was set, the abovementioned shear stress will be stronger than the platelet adhesion force, causing a platelet or a section of an aggregate to detach from the cluster. The mathematical equation for the platelet detachment status in Grade 1 can be described as below.

$$\delta > a$$

Here $\delta$: Distance between platelets derived from calculation

$\alpha$: Value used for determining detachment (set value) (preferably approximately 0.2$\mu$m)

Grade 2:

$$K_2 = \alpha \cdot K_1$$

$\alpha$ is a positive constant, preferably approximately $\alpha$=10.

$$\mu_2 = 2\sqrt{mK_2}$$

In Grade 2, adhesion occurs in a plurality of molecule sets transmitted by GPIb$\alpha$, producing intracellular signaling. In Grade 2, a coefficient is multiplied by the adhesion force calculated in Grade 1 and adhesion by a plurality of molecule sets is simulated. In Grade 2, the equation for the platelet detachment status can be described as below.

$$\delta > a$$

Here $\delta$: Distance between platelets derived from calculation

$\alpha$: Value used for determining detachment (set value) (preferably approximately 0.2$\mu$m)

Grade 3:

$$K_3 = \beta + K_2$$

$\beta$ is a positive constant, preferably approximately $\beta$=500.

$$\mu_3 = 2\sqrt{mK_3}$$

In order to simulate the steric structure of the GPIIb/IIIa activation state with the weakest adhesion force, a linear spring is added to the Grade 2 adhesion force. In Grade 3, the equation for the detachment status can be described as below.

$$\delta > a$$

Here $\delta$: Distance between platelets derived from calculation

$\alpha$: Value used for determining detachment (set value) (preferably approximately 0.2$\mu$m)

Grade 4:

$$K_4 = \gamma + K_2$$

$\gamma$ is a positive constant, preferably approximately $\gamma$=4000.

$$\mu_4 = 2\sqrt{mK_4}$$

In order to simulate the steric structure of the intermediate adhesion force of the GPIIb/IIIa activation state, a linear spring is applied in addition to the Grade 2 adhesion force.
Here the relationship between $\beta$ and $\gamma$ is $\beta < \gamma$.
In Grade 4, the equation for platelet detachment status can be described as below.

$$\delta > a$$

Here $\delta$: Distance between platelets derived from calculation

$\alpha$: Value used for determining detachment (set value) (preferably approximately 0.2$\mu$m)

Grade 5:

$$K_5 \approx \infty$$

$$\mu_5 = 2\sqrt{mK_5}$$

The equation was written so that an extraordinarily strong adhesion force can be applied because it is assumed that in Grade 5 platelets will not separate from the adhesion surface under any flow conditions.
In addition, the spring coefficient ($K_c$) and the viscosity coefficient ($\mu_c$) due to contact with collagen are defined as below.

$$K_c = D \cdot \sqrt{S}$$

$$\mu_c = 2\sqrt{mK_c}$$

Here, D is a constant.

It is known that there are portions of the activation from one Grade to another Grade that are reversible reactions, and other portions that are irreversible reactions. Both reversible reactions and irreversible reactions can also be recreated within the algorithm. The process for the recreation is as below.

1) if the distance between a Grade 1 or Grade 2 platelet and vWF exceeds the predetermined distance or,
2) if the distance between a Grade 1 or Grade 2 platelet and an activated platelet exceeds the predetermined distance, determine that the regressive reaction between Grade 1 and Grade 0 or Grade 2 and Grade 1 is reversible and return the activation state and spring coefficient settings to those of the lower Grade.

The setting for the amount of time it takes for each Grade to proceed to the next step can be modified at will, and this time parameter can be input in advance after data from actual biochemical experiments is referenced. Changes related to adhesion force between grades, in other words, the adhesion force at the time of change between the predetermined Grade and another Grade, can be expressed by changing the spring coefficient according to the Grade.
Specifics are as below.

Grade 0→Grade 1:

The change occurs when the number of collisions with vWF or the activated platelet ($T_1$) is as below.

$$T_1 > T_{1,crit}$$

Here, $T_{1,crit}$ is the input value, for example 0.1-10.
The input value is the criteria for the number of collisions between platelets that must be met or exceeded for the platelets to be activated to the following Grade. This value is input by a user using calculation condition setting means 911 (explained below) based on knowledge obtained from experiment results, etc.

Grade 1→Grade 2:

The change occurs when the relationship involving the activated platelet and adhesion time ($T_2$) is as below.

$$T_2 > T_{2,crit}$$

Here, $T_{2,crit}$ is the input value. If the adhesion time meets or exceeds this value when the platelet is in Grade 1, then the platelet is activated to the next Grade.
This value is input by a user using calculation condition setting means 911 based on knowledge obtained from experiment results, etc. $T_{2,crit}$ is, for example, 0.1-1 second.

Grade 2→Grade 3:

The change occurs when the relationship involving the activated platelet and adhesion time ($T_3$) is as below.

$$T_3 > T_{3,crit}$$

Here, $T_{3,crit}$ is the input value, and if the adhesion time meets or exceeds this value when the platelet is in Grade 2, then the platelet is activated to the next Grade.
This value is input by a user using calculation condition setting means 911 based on knowledge obtained from experiment results, etc. $T_{3,crit}$ is, for example, 0.1-10 seconds.

Grade 3→Grade 4:

The change occurs when the relationship involving the activated platelet and adhesion time ($T_4$) is as below.

$$T_4 > T_{4,crit}$$

Here, $T_{4,crit}$ is the input value, and if the adhesion time meets or exceeds this value when the platelet is in Grade 3, then the platelet is activated to the next Grade.

This value is input by a user using calculation condition setting means 911 based on knowledge obtained from experiment results, etc. $T_{4,crit}$ is, for example, 0.1-10 seconds.

Grade 4→Grade 5:

The change occurs when the relationship involving the activated platelet and adhesion time ($T_5$) is as below.

$$T_5 > T_{5,crit}$$

Here, $T_{5,crit}$ is the input value, and if the adhesion time meets or exceeds this value when the platelet is in Grade 4, then the platelet is activated to the next Grade.

This value is input by a user using calculation condition setting means 911 based on knowledge obtained from experiment results, etc. $T_{5,cirt}$ is, for example, 0.1-10 seconds.

As above, in the simulator of the present invention, the adhesion force at the time of change between the predetermined Grade and another Grade can be calculated, and actual platelets under a variety of conditions can be simulatedly shown. Accordingly, when the simulator of the present invention is used, it becomes possible to create a custom platelet simulation that reflects the platelet properties of a specific animal (including humans).

(3) Platelet Thrombus Simulator using the above Mathematical Model

The simulator of the present invention is equipped with the following means:

(a) a means for selecting, from previously stored equations, an equation for the calculation of adhesion force according to platelet activation grade;
(b) a means for calculating the abovementioned platelet adhesion force based on the abovementioned equation depending on the activation grade of each platelet;
(c) a means for outputting the coagulation status of platelets based on the calculated adhesion force of each abovementioned platelet.

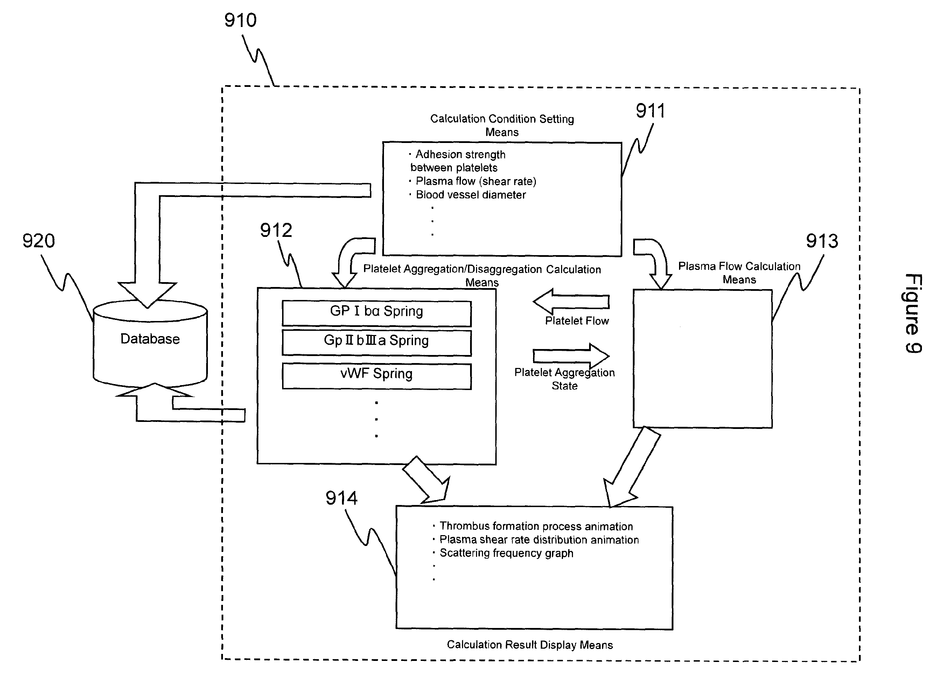

Fig. 9 is a structural diagram of the simulator of the present invention. In Fig. 9, the simulator of the present invention comprises Calculation Means 910 and Database 920, wherein Calculation Means 910 is equipped with (i) Calculation Condition Setting Means 911, (ii) Platelet Aggregation/Disaggregation Calculation Means 912, (iii) Plasma Flow Calculation Means 913, and (iv) Calculation Result Output Means 914.

(i) Calculation Condition Setting Means 911

Calculation Condition Setting Means 911 is a means for inputting, by means of a GUI (Graphical User Interface), conditions necessary for calculation input from a mouse or keyboard. Information that was input can be verified in the form of a graph.

In the present invention, the calculation condition setting means makes it possible to preliminarily store the abovementioned calculation equation that corresponds to platelet grade. The calculation equation uses parameters related to thrombus formation. Examples of these parameters include platelet density, concentration of platelets in the blood, platelet diameter, platelet concentration, platelet distribution ratio inside the blood vessel, spring coefficient, blood vessel diameter, blood vessel length, artery and vein classification, blood flow rate, size or shape of injured location, and type and ratio of the adhesion molecules (for example, a plurality of adhesion molecules with differing adhesion forces) expressed at the injured location. Parameters, are not, however, limited to the above and can be appropriately set depending on the purpose of the simulation. Here, the platelet distribution ratio inside the blood vessel means the ratio of the density of platelets flowing in close proximity to the blood vessel wall to the density of platelets whose flow is centered on the long axis of the blood vessel. Arteries and veins can be distinguished based on the presence or absence of pulse waves in the bloodstream.

Parameters related to thrombus detachment can also be used in the present invention. Examples of parameters related to thrombus detachment are platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location. Parameters related to thrombus detachment are not limited to the above.

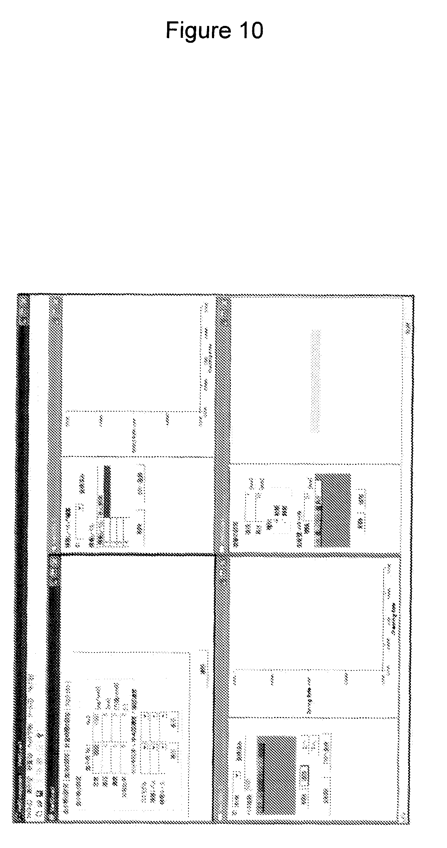

Fig. 10 shows an example of the input screen. Input conditions (parameters, calculating formula equation, etc.), are saved in database 920 and can be referenced again and reused.

(ii) Platelet Aggregation/Disaggregation Calculation Unit 912

Platelet aggregation/disaggregation calculation unit 912 is a means for calculating platelet adhesion force. Platelet aggregation/disaggregation calculation unit 912 selects the calculation equation from calculation condition setting means 911 or database 920 in order to calculate the adhesion force appropriate for the platelet activation grade and calculates platelet adhesion force based on the calculation equation appropriate for a platelet in each grade. With this means, platelet movement, adhesion, detachment, and scattering can be calculated. As springs for displaying adhesion force, for example, a GPIbα spring, a GPIIb/IIIa spring, a GPVI spring, a vWF spring, etc. can be positioned.

As stated above, shear rate increases linearly as plasma flow increases. In the present calculation unit, shear stress calculations based on the fluid force applied to a single platelet can be made using the below parameters.

Parameters: platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.

These parameters are converted into the following numerical values in order to calculate shear stress. Parameters are, however, not limited to these numerical values.

Platelet density: approximately 1000kg/m$^3$
Platelet diameter: approximately 1 to 2μm
Platelet concentration: 0 to 2 million platelets/μL
Platelet distribution ratio: Select from uniform distribution within blood vessel/concentration at the blood vessel wall
Spring coefficient: value derived from the above-described equation; approximately 200N/mm when plasma shear rate is approximately 1000/s
Blood vessel diameter: approximately 40μm to several mm
Blood vessel length: approximately 10 times the diameter
Artery and vein classification: in arteries, platelets are concentrated in close proximity to the blood vessel wall; in veins, platelets are uniformly distributed throughout the blood vessel
Blood flow rate: approximately 1mm/s to several hundred mm/s
Size or shape of injured location: one side is a rectangular shape approximately the size of the blood vessel diameter
Type and density of adhesion molecules at the injured location: vWF and collagen are positioned 1μm apart.

Shear stress is calculated by plugging the above parameters, which have been converted into numerical values, into the calculation equation. Calculation examples are given below.

Shear stress is calculated by multiplying the blood shear rate by the plasma viscosity coefficient. Platelet aggregation, however, changes the blood shear rate. Blood sheer rate is successively calculated by inputting the above parameters to calculate the platelet aggregation state and then, using the fluid force exerted by plasma flow on a single platelet as the reactive force acting on plasma flow, recalculating motion dispersion in order to solve for the changes in flow caused by platelet aggregates.

The motion of individual platelets can be expressed as shown below.

$$M\frac{d^2u}{dt^2} + C\frac{du}{dt} + Ku = f_x \quad \cdots\cdots (1)$$

$$M \frac{d^2 v}{dt^2} + C \frac{dv}{dt} + Kv = f_y \quad \cdots\cdots \quad (2)$$

$$M \frac{d^2 w}{dt^2} + C \frac{dw}{dt} + Kw = f_z \quad \cdots\cdots \quad (3)$$

Here,

$u$, $v$, $w$ : Each is a coordinate value in the three-dimensional space of the platelet.
$M$ : Platelet mass
$C$: Attenuation coefficient (the viscosity coefficient of the spring damper) (η in Fig. 8)
$K$ : Spring coefficient that expresses adhesion force
$f_x$, $f_y$, $f_z$: Express the force exerted by plasma flow in the x axis direction, y axis direction, and z axis direction, respectively, of the Euler coordinate system.

Platelet mass $M$ is solved in the following equation, which assumes that platelets are spherical in shape.

$$M = \rho \frac{4}{3} \pi \left( \frac{D}{2} \right)^3$$

Here,

ρ: Platelet density
D: Platelet diameter

Here, each parameter is used specifically in calculations as shown below.
Platelet density and platelet diameter are plugged into the equation for deriving platelet mass $M$, and the spring coefficient is plugged into the equation for deriving the above force exerted by plasma flow.
The classification of arteries and veins can be shown by using coordinate values $u$, $v$, *and* $w$ in a three-dimensional space. For arteries, coordinate values are assigned so that platelets concentrate and array themselves in close proximity to the vascular wall. For veins, coordinate values are assigned so that platelets are distributed uniformly (equally) in the blood vessel.
Platelet concentration is used to solve for the number of platelets that are generated inside the blood vessel. More specifically, by multiplying by the volume of the blood vessel for which platelet concentration is sought, the number of platelets that exist inside the blood vessel can be determined.
Platelet distribution ratio is used to set the classification between veins and arteries.
Spring coefficient is applied to $K$ in equations (1) to (3), which are used to solve for platelet motion.
Blood vessel diameter and blood vessel length are reflected as the space when determining the coordinates that indicate the position of each platelet.
Blood flow rate is used as the boundary condition when Navier-Stokes equations are solved; and size or shape of the injured location, in addition to type and density of the adhesion molecule at the injured location, are used as the boundary condition in equations (1) to (3) for the purpose of calculating platelet adhesion.
In addition, in the present invention, the adhesion force of the platelet can be calculated by converting the parameters into numerical values and plugging said numerical values into the equations.
Conversion of the parameters into numerical values can be performed, for example, as shown below. Numerical values are not, however, limited to the numerical values shown.

Platelet density: approximately $1000 kg/m^3$
Platelet diameter: approximately 1 to $2\mu m$

Platelet concentration: 0 to 2 million platelets/μL

Platelet distribution ratio: Select from uniform distribution within blood vessel/concentration at the blood vessel wall

Spring coefficient: value derived from the above-described equation; approximately 200N/mm when plasma shear rate is approximately 1000/s

Blood vessel diameter: approximately 40μm to several mm

Blood vessel length: approximately 10 times the diameter

Artery and vein classification: in arteries, platelets are concentrated in close proximity to the blood vessel wall; in veins, platelets are uniformly distributed throughout the blood vessel

Blood flow rate: approximately 1mm/s to several hundred mm/s

Size or shape of injured location: one side is a rectangular shape approximately the size of the blood vessel diameter

Type and density of adhesion molecules at the injured location: vWF and collagen are positioned 1μm apart.

An example of adhesion force calculation is given below.

Although the spring constant determines platelet adhesion force, the spring constant changes in accordance with the shear rate of the blood. Changes in adhesion force are computed by successively calculating plasma flow condition on the basis of results of platelet aggregation condition calculations according to the above parameters and subsequently computing changes in shear rate associated with platelet aggregate changes. In addition, adhesion force also changes when springs are positioned between platelets and a plurality of adhesion molecules. If the adhesion molecules at the injured location have a high density, the plurality of adhesion molecules and the springs will cause the platelets to bind, thereby increasing the adhesion force in an amount equivalent to the quantity of springs so used.

Here, each parameter is plugged into the predetermined calculation equation and used in calculations in the same way as described above.

Platelet density and platelet diameter are plugged into the equation for deriving platelet mass $M$ as $\rho$ and D, respectively.

Platelet concentration is used to solve for the number of platelets that are produced inside the blood vessel. In other words, by multiplying by the volume of the blood vessel for which platelet concentration is sought, the number of platelets that exist inside the blood vessel can be determined.

Platelet distribution ratio is used to set the classification between veins and arteries.

The spring coefficient is applied to $K$ in equations (1) to (3), which are used to solve for platelet motion.

The classification of arteries and veins can be shown by using coordinate values $u$, $v$, and $w$ in a three-dimensional space. For arteries, coordinate values are assigned so that platelets concentrate and array themselves in close proximity to the vascular wall. For veins, coordinate values are assigned so that platelets are distributed uniformly (equally) in the blood vessel.

Blood vessel diameter and blood vessel length are reflected as the space when determining the coordinates that indicate the position of each platelet.

Blood flow rate is used as the boundary condition when Navier-Stokes equations are omitted; and size or shape of the injured location, in addition to type and density of the adhesion molecule at the injured location, are used as the boundary condition in equations (1) to (3) for the purpose of calculating platelet adhesion.

(iii) Plasma Flow Calculation Means 913

Plasma Flow Calculation Means 913 is a means for calculating the motion of plasma flow based on platelet distribution.

In order to model the space involved in the plasma flow simulation, a section of the endovascular space that contacts coagulated platelets is divided into blocks to form a space section. The space section that was thusly formed is then divided into a grid shape to form multiple grid sections. The size of the divided grid sections can be configured as desired, and the dimensions of each section can be partially changed. Grid sections can be shaped into regular hexagon, cuboid, hexahedron, triangular pyramid, quadrangular pyramid, or triangular pole shapes, and the space section can also be divided into sections that are combinations of these various shapes. The wide variety of grid shapes that constitute the space are appropriately determined taking into account the platelet coagulation state and plasma flow etc. in the simulation.

After modeling the space section and grid sections as described above, the simulation program directs the plasma to flow into one end of the blood vessel, pass through the blood vessel, and flow out the other end of the blood vessel. This motion related to plasma flow can be shown by using the sequence of equations equivalent to the law of conservation of mass for general object motion and the Navier-Stokes equations equivalent to the law of conservation of momentum for general object motion shown below.

Accordingly, in the thrombus formation simulator of the present invention, the above plasma flow can be solved as shown below in the plasma flow computation unit.

The calculation equation is as shown below.

$$\rho \frac{\partial u_i}{\partial t} + \frac{\partial \rho u_i u_j}{\partial x_j} = \frac{\partial \sigma_{ij}}{\partial x_j} + F_i \quad \cdots \cdots \quad (4)$$

$$\sigma_{ij} = -\delta_{ij} p + \mu \left( \frac{\partial u_i}{\partial x_j} + \frac{\partial u_j}{\partial x_j} \right)$$

$$\frac{\partial \rho}{\partial t} + \frac{\partial \rho u_i}{\partial x_i} = 0 \quad \cdots \cdots \quad (5)$$

($i, j$ = 1,2,3)

Here, equations (4) and (5) are given in tensor form.

$u$ : Plasma flow speed
$\rho$ : Plasma density
$\mu$ : Plasma viscosity coefficient
$\delta_{ij}$ : Kronecker delta
$p$ : Plasma pressure
$F$ : Force exerted from platelet
$t$ : Time

In addition, $\sigma_{ij}$ ($i, j$ = 1, 2, 3) can be expressed by the 9 numerals in a three-dimensional coordinate system through the 3 column, 3 row mathematical expression below.

$$\sigma 1\,1 \quad \sigma 1\,1 \quad \sigma 1\,3$$

$$\sigma 2\,1 \quad \sigma 2\,2 \quad \sigma 2\,3$$

$$\sigma 3\,1 \quad \sigma 3\,2 \quad \sigma 3\,3$$

In the simulation of the present invention, plasma flow is analyzed by computing plasma flow in each of the grid sections located inside the endovascular section. Equations (4) and (5) above are used in these computations. The above equations (4) and (5) are discretely computed in accordance with the fact that the endovascular section is divided into grid sections.

More specifically, the equation is solved discretely using, for example, the finite volume method in a three-dimensional space ($x_1, x_2, x_3$) defined by blood vessel diameter and blood vessel length established by calculation condition setting (i) with plasma flow as an unknown number.

However, computations are not limited to the finite volume method. The finite difference method, boundary element method, finite element method, etc. can be selected as appropriate and performed in consideration of the simulation conditions.

Plasma density and the viscosity coefficient are set by (i) the Calculation Condition Setting Means. F, which is the force exerted on the platelet, is equivalent to the reaction force of the fluid force exerted on the platelet. F is solved through calculations performed by (ii) the Platelet Aggregation/Disaggregation Calculation Unit.

The simulation in the present invention shows the relationship between platelet adhesion force and plasma flow. Accordingly, plasma flow and platelet thrombus formation can be simulated by simultaneously solving equations (1) to (3) in addition to equations (4) and (5). For example, the motion of the gas flow in the entire space can be quantified by successively calculating the force applied by each element of motion related to specific-time plasma flow (plasma flow speed, flow direction, and plasma flux) for each short-time interval dt at the intersection of each grid section and compiling the calculation results for each intersection.

The finite volume method can be calculated by improving ITOCHU TechnoSolutions Corporation FINAS/CFD, etc.

Shear rate is computed from the calculated plasma flow, and the computed shear rate is reflected in the plasma adhesion force used in (ii) platelet aggregation/disaggregation calculation.

(iv) Calculation Result Output Means 914

Calculation output means 914 is a means for outputting the aggregation state of platelets based on the adhesion force for each platelet calculated as mentioned above and displaying platelet movement, aggregation, detachment, and scattering state calculated by (ii) above in the form of animation. Plasma flow shear rate calculated by (iii) can be displayed simultaneously.

Each numerical value related to blood flow motion or thrombus formation calculated as above can be visually displayed as the results of simulation by exclusive-use or general-purpose visualization software. For example, an isobaric line connecting plasma flows with identical pressure values or pressure distribution on an isobaric surface can be displayed, visually indicating various elements related to thrombus formation.

Further, a time series graph of scattering frequency according to activation level can be output.

(v) Data Storage Means

The input calculation conditions and calculation results are associated and, as a data storage means, saved in the database 920.

Stored calculation conditions and calculation results can be reread from the database 920 or the calculation condition setting means 911 and the calculation results display means 914.

3. Program for Simulating Platelet Thrombus Formation

The program of the present invention is a program for simulating the formation of platelet thrombi, said program executing the following procedure in a computer:

(a) selecting, from previously stored equations, an equation for the calculation of adhesion force according to platelet activation grade;
(b) calculating the abovementioned platelet adhesion force based on the abovementioned equation depending on the activation grade of each platelet;
(c) outputting the coagulation status of platelets based on the calculated adhesion force of each abovementioned platelet.

(1) Structural Example

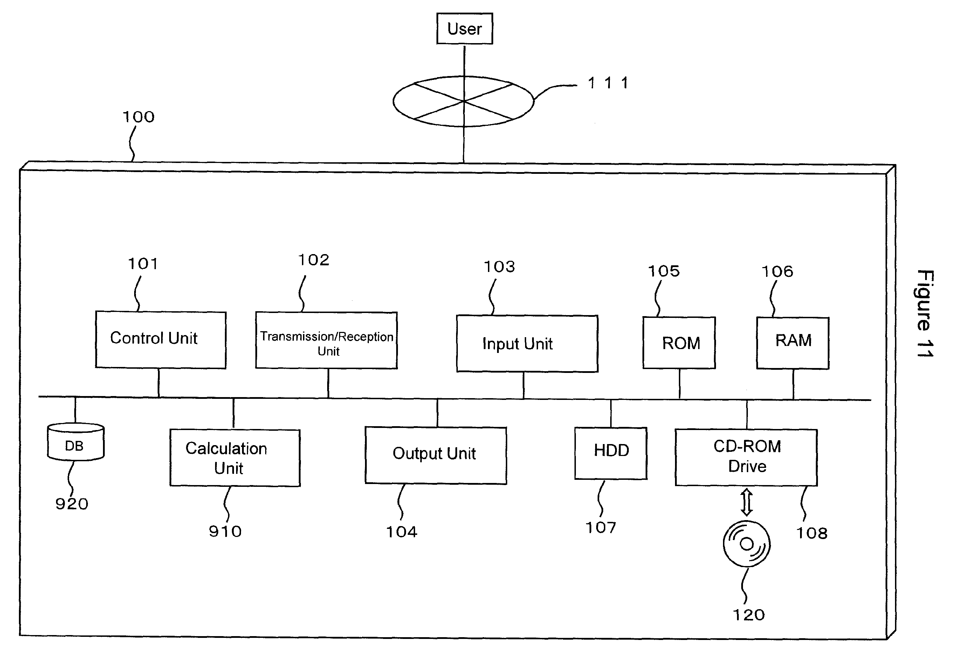

Fig. 11 shows a structural example of the means for controlling the computer in the program of the present invention.

Fig. 11 is a detailed structural diagram of a System 100, which implements the program of the present invention. In Fig. 11, the system 100 is equipped with a calculation unit 910 and a database (hereinafter DB) 920, which are shown in Fig. 9, and further comprises a control unit 101, a transmission/reception unit 102, an input unit 103, an output unit 104, a ROM 105, a RAM 106, a hard disk drive (hereinafter "HDD") 107, and a CD-ROM drive 108.

The Control Unit 101 1 is the central processing unit containing the CPU and MPU etc., and controls the entirety of the functions of system 100, in particular controlling the communications of the transmission/reception unit 102, or using data stored in the DB 920 to read display data for the platelet thrombus formation process and results thereof.

The transmission/reception unit 102 sends and receives data to and from the user terminal in accordance with instructions from the system 101. The user terminal can be connected via the internet cable 111. The transmission/reception unit 102 sends parameters and equations required for platelet thrombus formation processing, etc. to calculation unit 910.

The input unit 103 is a keyboard, mouse, touch panel, etc., and is operated to input parameters, when modifying the content of the DB920, etc. The output unit 104 is a LCD (liquid crystal display) etc., that converts the code data from the control unit 101 into display data each time the content of the DB 920 is modified, etc., and displays said display data. The ROM 105 stores the processing program of the system 100. The RAM 106 temporarily stores the data required by the system 100 for processing. The HDD 107 stores the program, etc., reads the stored program or data, etc. in accordance with instructions from the system 101, and stores said program or data, etc. in, for example, the RAM 106. The CD-ROM drive 108, in accordance with instructions from the system 101, reads the program, etc. stored on the CD-ROM 120, and writes said program, etc. to the RAM 106, etc. A rewritable recording medium such as a CD-R, CD-RW, etc. may be used instead of the CD-ROM 120. If a rewritable recording medium such as a CD-R, CD-RW, etc. may be used instead of the CD-ROM 120, a CD-R or CD-RW drive must be installed instead of the CD-ROM drive 108. Also, a medium such as DVD, magnetooptic disc, flash memory may be used in addition to the above media, if a structure with a compatible drive is employed.

(2) EXECUTION PROCEDURE

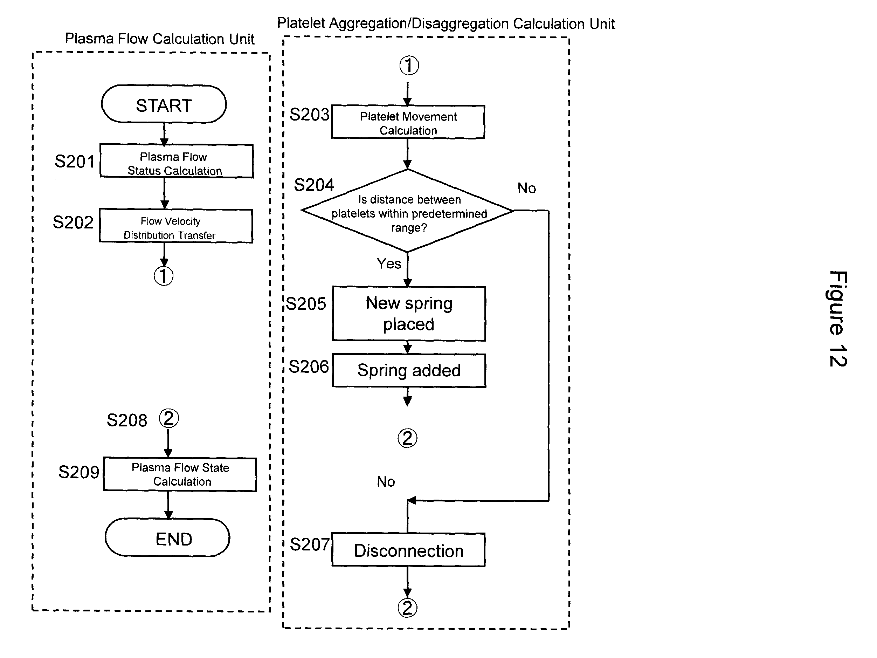

Fig. 12 is a flowchart explaining the workings of the program of the present invention.

The user uses calculation condition setting means 911 to input calculation conditions. When performing input, conditions previously registered and stored in the DB920 can be referenced/modified and then used.

The program of the present invention performs the following calculations. based on the inputted conditions.

(i) calculations by the platelet aggregation/disaggregation calculation means; and
(ii) calculations by the plasma motion calculation means

Calculations can be started based on an order from the control unit 101.

Calculations are performed in the order below.

(a) First, the plasma flow state in the blood vessel is calculated (S201), then the plasma flow state is transferred to the plasma aggregation/disaggregation calculation means in the form of a three-dimensional flow velocity distribution (S202).
(b) In the platelet aggregation/disaggregation computation means, platelet movement is calculated based on three-dimensional flow distribution (S203). If, at that time, either interplatelet distance or the distance between the platelet and the blood vessel is measured (S204), and the platelet is at or below the designated distance between another platelet or the injured section of the blood vessel, (S204, Yes), a spring corresponding to the platelet activation state and the shear rate at the location of the platelet is newly positioned (S205). The newly-positioned spring has the predetermined value that is incorporated into the equation in order to calculate the adhesion force corresponding to the platelet grade. The newly-placed spring strengthens when the shear stress increases and weakens when the shear stress decreases. Furthermore, springs are sequentially added in accordance with platelet adhesion time (activation) (S206).

In addition the spring placed between platelets is disconnected (S207) when the distance between platelets exceeds the predetermined distance (S204, No).

Here, the flowchart in Fig. 12 shows a calculation means for computing the adhesion force, coagulation, etc. related to a specific platelet when the platelet is used as a guideline. When another platelet (hereinafter "platelet 2") adheres to this platelet (hereinafter "platelet 1"), the adhesion force, aggregation, etc. related to platelet 2 can be calculated by using the flowchart in Fig. 12 in the same way that calculations for platelet 1 were performed. Accordingly, in the present invention, calculations are performed according to the process shown in Fig. 12 the same number of times as the number of platelets that appear in the simulation. Accordingly, adhesion force and aggregation, etc. are calculated in the same way as they were calculated for platelet 1 and platelet 2 for either one or both of platelet 1 and platelet 2, or for when a different platelet (platelet 3, platelet 4, etc.) adheres thereto.

Regarding additional springs, the distance between platelets is also measured (S204).

(c) The position and velocity of platelets calculated by the platelet aggregation/thrombus disaggregation calculation means is transferred to the plasma flow calculation means (S208). In the plasma flow calculation means, plasma flow state is calculated based on platelet position and velocity (S209). When platelets are aggregated or stationary, and a flow state that allows the plasma to circumvent the aggregates is calculated.
(d) The calculations in (a) to (c) above are repeated alternately until the predetermined time is reached. According to these calculations, the control unit 101 outputs the aggregation state of the platelets. "Aggregation state" as used herein means either platelets aggregating within a specific range of time or the aggregated condition of platelets at a specific time.
(e) When the calculations in (d) above are repeated and platelets aggregate, shear stress caused by plasma flow is exerted on aggregates. Based on this shear stress, the program of the present invention outputs the detachment state of platelets. Regarding the aggregation of platelets, each of the calculations shown in Fig. 12 is performed on each platelet as time passes. Accordingly, control unit 101 contrasts the entirety of the flowchart shown in Fig. 12 or a part thereof with plasma flow, and, when a specified shear stress is generated, calculates which spring corresponding to which part of the platelet aggregate to disconnect. In other words, control unit 101 calculates how large an aggregate to detach or allow to "fly", and outputs the platelet detachment state. "Detachment state" as used herein means either a platelet detaching within a specific range of time or the detached condition of a platelet at a specific time. Therefore, the detachment state of the above spring, in other words, the platelet detachment state (aggregate size) can be the entirety or a part of the platelet aggregate. There are instances in which only the platelets on the surface of the aggregate detach and instances in which a section of the aggregate peels off.

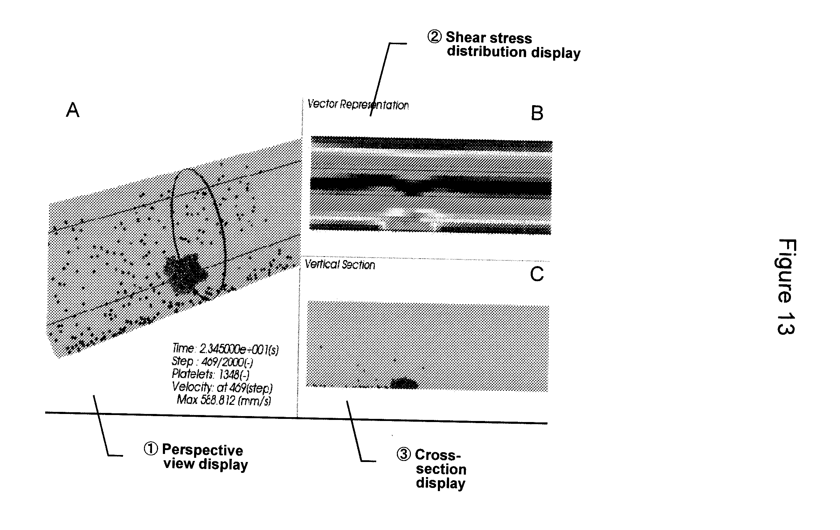

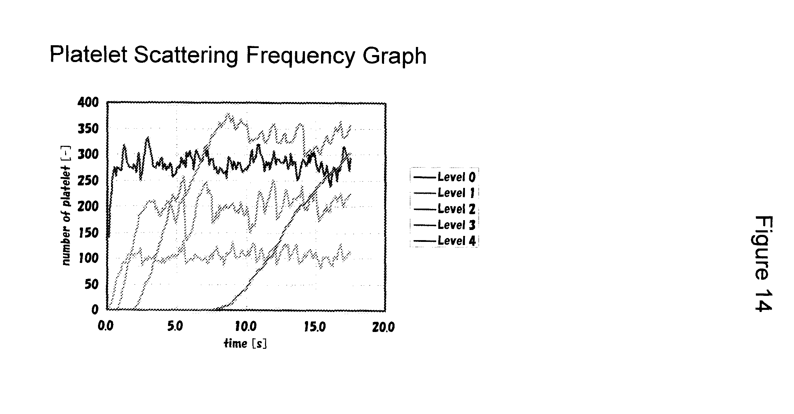

Then, the calculated results are displayed using calculation result display means 914. Figs. 3, 4, 13, and 14 show an example of display results. Fig. 13 shows a manner in which a blood vessel perspective view (bird's-eye-view, panel A), a shear stress distribution chart (panel B), and a blood vessel longitudinal cross-section diagram (panel C) are displayed on a single screen, while Fig. 14 shows a manner in which platelet scattering frequency is displayed as a graph.

Calculation results are successively saved in the database of the (v) Data Storage Means.

4. Computer-readable Recording Media

The program of the present invention can be written in a computer language such as C, Java, Perl, Fortran, or Pascal and is configured to be cross-platform applicable. Accordingly, the software can run on Windows ™ 95/98/2000/XP, Linux, UNIX ™, or Macintosh.

The program of the present invention can be stored on a computer-readable recording medium or on a storage means that can be connected to a computer. The computer-use recording medium or storage means that contains the program of the present invention is included in the present invention. Recording media or storage means include, but are not limited to, magnetic media (flexible disk, hard disk, etc.), optical media (CD, DVD, etc.), magnetic optical media (MO, MD), and flash memory.

The present invention is described in detail through embodiments below with the proviso that the present invention is not limited to the embodiments.

Example 1

*In vitro* human platelet and vWF solid-phase surface adhesion state analysis in a parallel plate micro-fluid channel

In this example, platelet thrombus formation was simulated under the following condition settings:

| | |
|---|---|
| Platelet diameter | 1 ($\mu$m) |
| Shear stress | 100 to 3,000 (1/s) |
| Blood vessel diameter | 40 ($\mu$m) |
| Blood vessel length | 400 ($\mu$m) |
| Phenomenon duration | 3 (minutes) |

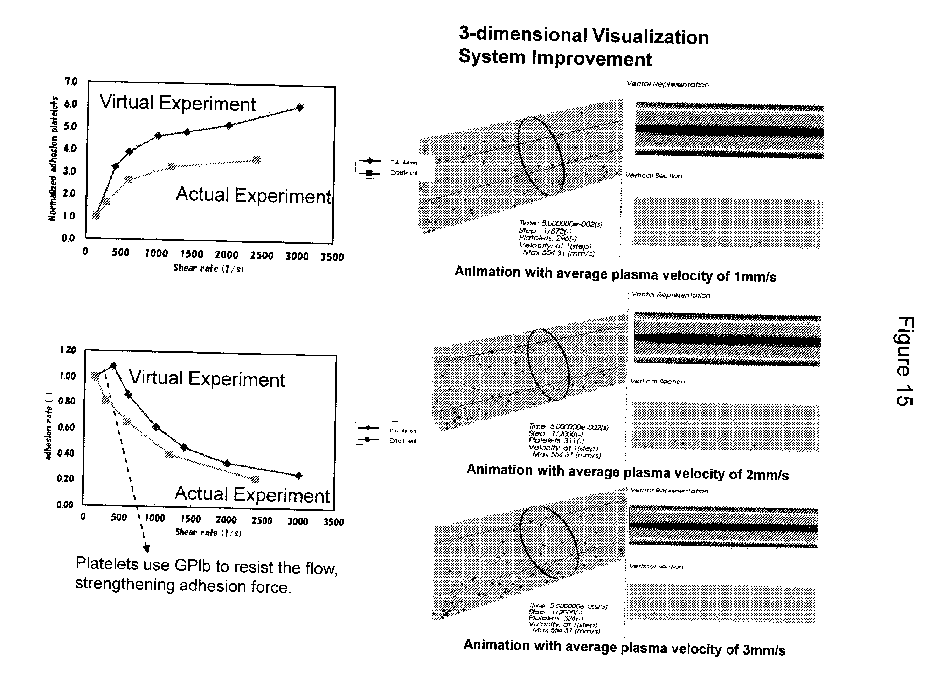

The results are shown in Fig. 15.

Fig. 15 shows that when a simulation is conducted using the simulator of the present invention, there is strong coordination with actual measured values, showing that actual platelet thrombus formation can be simulated

Industrial Applicability

The present invention provides a platelet thrombus formation simulator for simulating *in vivo* platelet formation. According to the present invention, the breakdown and flow of thrombi, which is the cause of cerebral infarction, can be accurately reproduced in blood vessels. Therefore, according to the present invention, medicines that efficiently and safely remove thrombi can be predicted, thereby making the present invention extremely useful in the selection of medicines for patient treatment.

**Claims**

1. A platelet thrombus simulator equipped with the following means:

   (a) a means for selecting, from previously stored equations, an equation for the calculation of adhesion force according to platelet activation grade;
   (b) a means for calculating the platelet adhesion force based on the equation depending on the activation grade of each platelet;
   (c) a means for outputting the coagulation status of platelets based on the calculated adhesion force of each platelet.

2. The simulator according to claim 1, wherein the simulator further comprises the procedure in which the shear stress exerted on the surface of a clump of aggregated platelets is calculated using the inputted parameters related to platelet thrombus detachment, and the state of platelet detachment from said platelet aggregate based on said shear stress is output.

3. The simulator according to claim 1, wherein the calculation equation uses the parameters related to thrombus formation.

4. The simulator according to claim 3, wherein the parameters are at least one of the following selected from the group consisting of: platelet concentration in the blood, platelet diameter, platelet concentration, platelet distribution ratio, spring coefficient, blood vessel diameter, blood vessel length, artery and vein classification, blood flow rate, size or shape of injured location, and type and ratio of the plurality of adhesion molecules expressed at the injured location.

5. The simulator according to claims 1 or 2, wherein output of the platelet aggregation state and/or detachment state is output to the predetermined display means.

6. The simulator according to claim 5, wherein the display means displays at least one of the following selected from the group consisting of: an animated display of the platelet coagulant status and/or detachment status; a display of the activity level of individual platelets constituting the platelet aggregates; an animated display of platelet shear rate distribution; a display of the platelet aggregate scattering frequency and size of individual aggregates; and a display in graph form of the activity level of individual platelets contained in scattered aggregates.

7. The simulator according to claims 1 or 2, wherein the display output is at least one of the following selected from the group consisting of: blood vessel coagulation status and/or detachment status in a perspective view; blood vessel coagulation status and/or detachment status in a longitudinal cross-section diagram; and blood vessel longitudinal shear stress in a distribution diagram.

8. The simulator according to claim 2, wherein the parameters related to platelet detachment are at least one of the following selected from the group consisting of: platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.

9. A program for the simulation of platelet thrombus formation, said program executing the following procedure in a computer:

   (a) selecting, from previously stored equations, an equation for the calculation of adhesion force according to platelet activation grade;
   (b) calculating the platelet adhesion force based on the equation depending on the activation grade of each platelet;
   (c) outputting the coagulation status of platelets based on the calculated adhesion force of each platelet.

10. The program according to claim 9, wherein the program further comprises the procedure in which the shear stress exerted on the surface of a clump of aggregated platelets is calculated using the inputted parameters related to platelet thrombus detachment, and the state of platelet detachment from said platelet aggregate based on said shear stress is output.

11. The program according to claim 9, wherein the equations use parameters related to thrombus formation.

**12.** The program according to claim 11, wherein the parameters are at least one of the following selected from the group consisting of: platelet concentration in the blood, platelet diameter, platelet concentration, platelet distribution ratio, spring coefficient, blood vessel diameter, blood vessel length, artery and vein classification, blood flow rate, size or shape of injured location, and type and ratio of the plurality of adhesion molecules expressed at the injured location.

**13.** The program according to claims 9 or 10, wherein output of the aggregation state and/or detachment state of the platelets is to a predetermined display means.

**14.** The program according to claim 13, wherein the display means displays at least one of the following selected from the group consisting of: an animated image of the platelet coagulant status and/or detachment status; a display of the activity level of individual platelets constituting the platelet aggregates; an animated display of platelet shear rate distribution; a display of the platelet aggregate scattering frequency and size of individual aggregates; and a display in graph form of the activity level of individual platelets contained in scattered aggregates.

**15.** The program according to claims 9 or 10, wherein output from the output means is a display output of at least one of the following selected from the group consisting of: blood vessel coagulation status and/or detachment status in a perspective view; blood vessel coagulation status and/or detachment status in a longitudinal cross-section diagram; and blood vessel longitudinal shear stress in a distribution diagram.

**16.** The program according to claim 10, wherein the parameters related to platelet detachment are at least one of the following selected from the group consisting of: platelet density, platelet diameter, platelet concentration, platelet distribution ratio, spring coefficient, blood vessel diameter, blood vessel length, artery and vein classification, blood flow rate, size or shape of injured location, and type and density of adhesion molecules at the injured location.

**17.** A computer-readable recording medium on which the computer program according to any one of claims 9 to 16 is stored.

# Figure 1

# Figure 2

# Figure 3

# Figure 4

Figure 5

Figure 6

# Figure 7

# Figure 8

Figure 9

**910**

**911**

Calculation Condition Setting Means

· Adhesion strength between platelets
· Plasma flow (shear rate)
· Blood vessel diameter
·
·
·

**920**

**912**

Database

Platelet Aggregation/Disaggregation Calculation Means

GP I bα Spring

Gp II bIIIa Spring

vWF Spring

·
·
·

Platelet Flow

Platelet Aggregation State

**913**

Plasma Flow Calculation Means

**914**

· Thrombus formation process animation
· Plasma shear rate distribution animation
· Scattering frequency graph
·
·

Calculation Result Display Means

EP 2 075 754 A1

30

# Figure 10

Figure 11

Plasma Flow Calculation Unit

Platelet Aggregation/Disaggregation Calculation Unit

START

S201 | Plasma Flow Status Calculation

S202 | Flow Velocity Distribution Transfer

①

S208 ②

S209 | Plasma Flow State Calculation

END

①

S203 | Platelet Movement Calculation

S204 | Is distance between platelets within predetermined range?

No

Yes

S205 | New spring placed

S206 | Spring added

②

No

S207 | Disconnection

②

Figure 12

# Figure 13

② Shear stress distribution display

B

Vector Representation

C

Vertical Section

③ Cross-section display

Time : 2.345000e+001(s)
Step : 469/2000(-)
Platelets: 1348(-)
Velocity: at 469(step)
Max 568.812 (mm/s)

① Perspective view display

A

Figure 14

Platelet Scattering Frequency Graph

Figure 15

## INTERNATIONAL SEARCH REPORT

| International application No. |
|---|
| PCT/JP2007/068481 |

A. CLASSIFICATION OF SUBJECT MATTER
*G06Q50/00* (2006.01) i

According to International Patent Classification (IPC) or to both national classification and IPC

B. FIELDS SEARCHED

Minimum documentation searched (classification system followed by classification symbols)
G06Q50/00

Documentation searched other than minimum documentation to the extent that such documents are included in the fields searched
Jitsuyo Shinan Koho        1922-1996    Jitsuyo Shinan Toroku Koho    1996-2007
Kokai Jitsuyo Shinan Koho  1971-2007    Toroku Jitsuyo Shinan Koho    1994-2007

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| A | Hiroki KAMADA, Ken'ichi TUBOTA, Shigeo WADA, Takami YAMAGUCHI, "Kesshoban no Gyoshu ni yoru Ichiji Kessen no Keisei·Hokai no Ryushiho Simulation", Transactions of the Japan Society of Mechanical Engineers (Series B), 25 May, 2006 (25.05.06), Vol.72, No.717, pages 1109 to 1115 | 1-17 |
| A | JP 2006-166974 A (National Institute of Advanced Industrial Science and Technology), 29 June, 2006 (29.06.06), Full text; Figs. 1 to 8 (Family: none) | 1-17 |

☐ Further documents are listed in the continuation of Box C.          ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>10 October, 2007 (10.10.07) | Date of mailing of the international search report<br>23 October, 2007 (23.10.07) |
|---|---|
| Name and mailing address of the ISA/<br>Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006250798 A

**Non-patent literature cited in the description**

- *Biorheology,* 2003, vol. 40, 265-272